# EUROPEAN PATENT APPLICATION

(11) **EP 1 714 675 A1**
(43) Date of publication of application: **25.10.2006**
(21) Application number: 05075951.3
(22) Date of filing: 21.04.2005
(51) Int. Cl.: A61P 17/02, A23K 1/18, A23K 1/16

(54) **Method for increasing the speed of wound healing of injured animals**

(71) Applicant: Desol BV, 6167 RA Geleen (NL)
(72) Inventor: Van den Elshout, Wilhelmus Hubertus Henricus A., 6167 RA Geleen (NL); Forier, Rudi Ludovicus Florent, 3520 Zonhoven (BE)
(74) Representative: Habets, Winand

(57) **Abstract**

This invention is in the field of animal food, in particular food for wounded animals. Surprisingly, it has now been found that the speed of recovery of wounded animals may be increased by feeding the animal with a certain amount of natural occurring immune enhancing ingredients such as beta-glucans and/or phytohormones like auxin or gibberellic acid. Also, combinations of these substances, in particular the combination of beta-glucans and phytohormones, were shown to have a synergistic effect in that they improved the recovery speed of the animal more than the individual components on their own.

## Description

This invention is in the field of animal food, in particular food for wounded animals. Food according to the invention increases the speed of wound healing of injured animals.

Wound healing is the process of repair that follows injury to the skin and other soft tissues. Symptoms of wound healing include swelling, stiffness, tenderness, discoloration, skin tightness, scabbing, itching, and scar formation.

Wounds may result from trauma or from a surgical incision. Wounds may also result from bone fracture or from giving birth. In addition, pressure ulcers (also known as decubitus ulcers or bed sores), a type of skin ulcer, might also be considered wounds. The capacity of a wound to heal depends in part on its depth, as well as on the overall health and nutritional status of the individual.

Following injury, an inflammatory response occurs and the cells below the dermis (the deepest skin layer) begin to increase collagen (connective tissue) production. Later, the epithelial tissue (the outer skin layer) is regenerated. Dietary modifications and nutritional and herbal supplements may improve the quality of wound healing by influencing these reparative processes or by limiting the damaging effects of inflammation.

Over the counter topical antibiotics, such as neosporin (Myciguent®), bacitracin (Baciguent®), and combinations of the two with polymyxin B (Neosporin®, Polysporin®) are used to treat skin infections and promote wound healing. Prescription strength topical antibiotics, such as metronidazole (MetroGel®) and mupirocin (Bactroban®), might be necessary to treat infection and promote healing.

Other treatment includes keeping the wound clean, dry, and covered. Surgical treatments, such as stitches and removal of damaged tissue, may be recommended.

Building and repairing tissue requires adequate amounts of calories and protein to fuel the repair mechanisms, as the skin and underlying tissues are made of protein. While major wounds from extensive injuries or major surgery significantly raise protein and calorie requirements, optimal healing of minor wounds should not require changes from a typical, healthful diet.¹ In a study of malnourished people with skin ulcers, those who were given a diet containing 24% protein showed a significant reduction in the size of the ulcer, whereas those given a diet containing 14% protein had no significant improvement.² This study suggests an increase in dietary protein can improve wound healing in malnourished people. It is not known whether the same benefit would be observed in well-nourished people.

Supplementation with bromelain, an enzyme derived from pineapple stem, prior to and following a surgical procedure has been shown to reduce swelling, bruising, healing time, and pain.³ Bromelain supplementation has also been shown to accelerate the healing of soft-tissue injuries in male boxers.⁴ The amount of bromelain used in these studies was 40 mg four times per day, in the form of enteric-coated tablets. Enteric-coating prevents the stomach acid from partially destroying the bromelain. Most currently available bromelain products are not enteric-coated, and it is not known if such products would be as effective as enteric-coated bromelain.

Thiamine (vitamin B1),⁵ pantothenic acid (vitamin B5),⁶ and other B vitamins⁷ have all been shown to play a role in wound healing in animal studies. For this reason, although human research is lacking, some alternative healthcare practitioners recommend a high-potency B vitamin supplement to promote wound healing.

Vitamin C is needed to make collagen (connective tissue) that strengthens skin, muscles, and blood vessels and to ensure proper wound healing. Severe injury appears to increase vitamin C requirements,⁸ and vitamin C deficiency causes delayed healing.⁹ Preliminary human studies suggest that vitamin C supplementation in non-deficient people can speed healing of various types of wounds and trauma, including surgery, minor injuries, herniated inter-vertebral discs, and skin ulcers.^{10 11} A combination of 1-3 grams per day of vitamin C and 200-900 mg per day of pantothenic acid has produced minor improvements in the strength of healing skin tissue. ^{12 13}

Zinc is a component of many enzymes, including some that are needed to repair wounds. Even a mild deficiency of zinc can interfere with optimal recovery from everyday tissue damage, as well as from more serious trauma.^{14 15} One controlled trial found the healing time of a surgical wound was reduced by 43% with oral supplementation of 50 mg of zinc three times per day, in the form of zinc sulfate.¹⁶

Preliminary²⁰ and controlled ²¹ studies of people with severe burns and other types of injuries²² showed that supplementation with 10-30 grams of ornithine alpha-ketoglutarate (OKG) per day significantly improved wound healing and decreased the length of hospital stays. Improved healing from major trauma and surgery has also been demonstrated with oral supplements including several grams per day of glutamine.23

Vitamin A plays a central role in wound healing,²⁴ but the effect of supplemental vitamin A in people who have suffered a minor injury and are not vitamin A-deficient remains unclear. Vitamin A supplements have been shown to improve healing in animal studies,²⁵ and may be especially useful in a topical ointment for skin injuries in people taking corticosteroid medications.²⁶ Although there are no studies in humans, some doctors recommend 25,000 IU of vitamin A per day, beginning two weeks prior to surgery and continuing for four weeks after surgery.

Animal studies have shown that supplementing with vitamin E can decrease the formation of unwanted adhesions following a surgical wound. In addition, wound healing was more rapid in animals fed a vitamin E-rich diet than in those fed a standard diet.²⁷ In another study, however, wound healing was inhibited by supplementation with a massive amount of vitamin E (equivalent to about 35,000 IU).²⁸ This adverse effect of vitamin E was prevented by supplementation with vitamin A. Although the relevance of these studies to humans is not clear, many doctors recommend supplementing with both vitamins A and E in order to enhance wound healing and prevent adhesion formation. Typical amounts recommended are 25,000 IU of vitamin A per day and 400 IU of vitamin E per day, beginning two weeks prior to surgery and continuing for four weeks after surgery.

Topical application of vitamin E is sometimes recommended for preventing or treating post-injury scars, although only three controlled studies have been reported. Two of these trials found no effect on scar prevention after surgery,^{29 30} and one trial found vitamin E improved the effect of silicon bandages on large scars called keloids.³¹

Copper is a required cofactor for the enzyme lysyl oxidase, which plays a role in the cross-linking (and strengthening) of connective tissue³² Doctors often recommend a copper supplement as part of a comprehensive nutritional program to promote wound healing. A typical amount recommended is 2-4 mg per day, beginning two weeks prior to surgery and continuing for four weeks after surgery.

Other trace minerals, such as manganese, copper, and silicon, are known to be important in the biochemistry of tissue healing.^{33 34 35 36} However, there have been no controlled trials exploring the effect of oral supplementation of these minerals on the rate of healing.

Glucosamine sulfate and chondroitin sulfate may both play a role in wound healing by providing the raw material needed by the body to manufacture connective tissue found in skin, tendons, ligaments, and joints.³⁷ Test tube and animal studies have found that these substances, and others like them, can promote improved tissue healing.^{38 39 40 41 42} One controlled trial in humans found that wounds healed with greater strength when they were treated topically with a chondroitin sulfate-containing powder.⁴³ However, no research has investigated the value of oral supplements of glucosamine or chondroitin for wound healing in humans.

Arginine supplementation increases protein synthesis and improves wound healing in animals.⁴⁴ Two trials have shown increased tissue synthesis in surgical wounds in people given 17-25 grams of oral arginine per day.^{45 46}

Carnosine is a small molecule composed of the amino acids histidine and alanine. The exact biological role of carnosine is not completely understood, but animal research demonstrates that it promotes wound healing.⁴⁷

While many herbs may be useful in wound healing, it is important that wounds be properly cleaned and dressed before any herbal preparations are applied. This will prevent infection.

In animal studies of skin inflammation, both topical and oral aloe vera have proven beneficial in decreasing inflammation and promoting cellular repair.^{48 49} Topical aloe vera has facilitated wound healing in controlled human research, as well.⁵⁰ In one controlled trial, however, topical aloe vera gel was inferior to conventional management of surgical wounds.⁵¹

One preliminary trial found that a gotu kola extract helped heal infected wounds (unless they had reached bone).⁵² A review of French studies suggests that topical gotu kola can help wounds.⁵³ One study found gotu kola extract helpful for preventing and treating enlarged scars (keloids).⁵⁴ Standardized extracts of gotu kola containing up to 100% total triterpenoids are generally taken, providing 60 mg once or twice per day. Animal studies have shown that constituents in gotu kola, called asiaticosides, increase antioxidant levels during wound healing and facilitate repair of connective tissues.^{55 56}

Horse chestnut contains a compound called aescin that acts as an anti-inflammatory and reduces edema (swelling with fluid) following trauma, particularly sports injuries, surgery, and head injury.⁵⁷ A topical aescin preparation is popular in Europe for the treatment of acute sprains during sporting events.

A topical preparation of chamomile combined with corticosteroids and antihistamines has been used to speed wound healing in elderly people with stasis ulcers caused by inadequate circulation,⁵⁸ as well as in people who had tattoos removed.⁵⁹ Topical use of chamomile ointment was also found to successfully treat mild stasis ulcers in elderly bedridden patients.⁶⁰

Topical application of honey has been used since antiquity to accelerate skin wound healing.⁶¹ Honey has been shown to inhibit the growth of several organisms responsible for wound infections.^{62 63 64} In one preliminary study, nine infants with large, open infected wounds that failed to heal with conventional treatment were treated successfully with topical application of honey.⁶⁵ Fresh unprocessed honey was applied to wounds in amounts of 5-10 ml twice daily for a period of 21 days. All infants showed marked clinical improvement after 5 days, and the wounds were closed and free of infection by 21 days. The use of honey to treat wounds should be supervised by a doctor.

Used topically, some practitioners consider arnica to be among the best vulnerary (wound-healing) herbs available.⁶⁶ Topical use of arnica is approved by the German government for improving wound healing.⁶⁷ Arnica is poisonous if taken internally.

Calendula flowers were historically considered beneficial for wound healing, reducing inflammation and fighting infection as a natural antiseptic.⁶⁸ Like echinacea, calendula is approved in Germany for use in treating poorly healing wounds.⁶⁹ Generally 1 tablespoon (15 grams) of calendula flowers is steeped in hot water for 15 minutes, then cloths are dipped into the liquid to make compresses. Such compresses should be applied for at least 15 minutes, initially several times per day, then tapering off as the wound improves.

Traditional herbalists sometimes recommend the topical use of herbs such as St. John's wort, calendula, chamomile, and plantain, either alone or in combination, to speed wound healing. Clinical trial in humans have not yet validated this traditional practice.

Echinacea is used among European practitioners of herbal medicine to promote wound healing⁷⁰ and is approved by the German government for this use.⁷¹ Creams or ointments are applied several times a day to minor wounds.

Comfrey has anti-inflammatory properties that may decrease bruising when the herb is applied topically.⁷² Comfrey is also widely used in traditional medicine as a topical application to help heal wounds.⁷³ Witch hazel can also be used topically to decrease inflammation and to stop bleeding.⁷⁴ Native Americans used poultices of witch hazel leaves and bark to treat wounds, insect bites, and ulcers.⁷⁵ Horsetail can be used both internally and topically to decrease inflammation and promote wound healing.⁷⁶

Despite all these products that may improve the speed of wound healing there is still room for alternative products that can play a role in this beneficial process.

Surprisingly, it has now been found that the speed of recovery of injured animals may be increased by feeding the animal with a certain amount of natural occuring immune enhancing ingredients such as beta-glucans and/or phytohormones like auxin or gibberellic acid. Also, combinations of these substances, in particular the combination of beta-glucans and phytohormones, were shown to have a synergistic effect in that they improved the speed of recovery of the animal more than these individual components on their own.

The term wounded animals or injured animals is used herein to refer to animals that have experienced a physical trauma, such as bone fractures, flesh wounds, internal wounds or have recently given birth or have undergone surgery.

Phytohormones are herein defined as molecules that function to coordinate plant growth and development. The compounds that have been considered as plant hormones are for instance: indole-3-acetic acid (auxin), cytokinin, gibberellin, ethylene, abscisic acid. In addition, brassinosteroids, jasmonic acid and salicylic acid have been shown to have important growth regulating activities and are considered to function as Phytohormones.

Particularly good results were obtained when the animal feed was supplemented with free IAA instead of conjugated IAA. Free IAA and conjugated IAA are known compounds, free IAA is a naturally-occurring plant growth phytohormone which has been extensively studied. In plants, most of the IAA occurs in a conjugated form (Slovin et al. 1999, Biochemistry and molecular biology of plant hormones, Elsevier, Amsterdam. P115-140), either conjugated to sugars via ester linkages or to amino acids and peptides via amide linkages.

Free IAA is readily available as a commercial product. It may be synthesised chemically or prepared in a biological way. IAA producing micro-organisms are widespread in nature. Yeast, fungi and many bacteria as well as plants are known to convert precursors of IAA into free IAA. In addition to the L-tryptophan conversion by bacteria, also L-tryptophan independent biochemical routes towards free IAA are described extensively (J. Plant Growth Regul (2001) 20: 198-216).

A well known bacterium, capable of producing free IAA is Azospirillum Brasilense (AB). At the end of the growth phase in a regular fermentation process, AB is able to convert L-tryptophan into free IAA. To increase the efficiency of this conversion, a small amount of synthetic free IAA may be added to the media. Via a feedback mechanism, AB increases the conversion of L-tryptophan into free IAA.

Final concentrations of 1 gram free IAA / liter culture broth are easy to make, but even much higher concentrations are possible, depending on the micro-organism used.

After ending the fermentation the micro-organism may be lysed and a powder enriched in free IAA may be obtained by spray drying or any other convenient way of drying the culture broth. Other techniques may be used to remove liquids partly or completely.

The term "free IAA" is used herein to indicate that the free IAA is in the free or acid form, whereas the term "conjugated IAA" refers to IAA that is conjugated via ester linkages or via amide linkages.

As long ago as 1956, the effects of free IAA on humans were studied, and it was shown that single doses of 0.1 g/kg body weight were non-toxic (Mirsky A and Diengott D, Hypoglycemic action of indole-3-acetic acid by mouth in patients with diabetes mellitus, Proc. Soc. Exp. Biol. Med. 93: 109-110,1956). In 1964, it was found that photo-oxidation products of free IAA acted as growth inhibitors of micro-organisms (Still C, Fukuyama T and Moyed H, Inhibitory Oxidation Products of Indole-3-acetic acid, J. Biological Chemistry, 240,6,2612-2618,1964).

Also, the medical use of free IAA and some of its derivatives has previously been described. EP 1.296.676 describes the use of free IAA as a pharmaceutical, in particular for treating neoplastic disease in humans. WO 02/080906 describes the use of free IAA for treating endometriosis in women. Nachson et al. (Feed and Chemical Toxocology 41, 745-752) reported the effect of some free IAA derivates (indole-3-carbinol and 3,3'-diindolylmethane) on the proliferation and induction of apoptosis in human prostate cancer cell lines whereas Rossiter et al. (Bioorganic & Medicinal Chemistry Letters, 12, 2523-2526) as well as Folkes et al. (Biochemical Pharmacology 63, 265-272) described the use of free IAA and some derivatives in enzyme-prodrug directed cancer therapies.

Phytohormones and beta-glucans appeared to work in a wide range of concentrations for improving the speed of wound healing in animals. The optimal concentrations may vary between different species, however, the skilled person will know how to obtain an optimal concentration for a given species, for instance by titration of the desired compound into the animal feed and testing when this would have the optimal effect. The following may serve as guidance in this process.

A skilled person will appreciate that the amount of free IAA in the ready to use feed has to be adjusted in order to supply the animal with an effective amount of free IAA. In order to adjust the free IAA concentration in the feed so that a certain daily intake of free IAA is achieved, an estimate has to be made of the feed intake of an animal or animal group. A skilled person is aware of the feed intake of a (particular kind or group of) animal(s), usually the feed intake per day is between 0,5 and 10% of the body weight of the animal, with occasional exceptions as high as 20 %. Elderly animals tend to eat less and are considered to have a feed intake per day between 0,1 and 5 %, typically of 1% of their body mass.

It was found that wounded animals recovered better and quicker from their injuries when free IAA was provided in their feed in the range of 0,004 and 40 mg per kilogram life weight per day (mg/kglw/day). Optimum between cost and benefit was reached in concentrations between 0,04 and 4 mg/kglw/day, in particular feed with 0,4 mg/kglw/day free IAA was very effective.

It was also found that, within the family of beta-glucans, in particular 1,3 and 1,6 beta glucans were very useful to improve the speed of recovery of wounded animals. A particularly good source of such 1,3 and 1,6 beta glucans may be found in preparations of Agaricus blazei murill or yeast cell walls. Animal feed supplemented with 1 to 1000 mg/kglw/day of dried Agaricus blazei murill was found to produce the desired effect of improving the speed of recovery of wounded animals. This corresponds to approximately 0,1 to 100 mg/kglw/day of pure 1,3 and 1,6 beta glucans. Excellent results were obtained with animal feed containing 1 to 10 mg/kglw/day of pure 1,3 and 1,6 beta glucans, optimum of cost benefit was found to be around 5 mg/kglw/day, corresponding to 50 mg/kglw/day of dried ABM.

The effect of improving the speed of recovery was also observed when the feed of injured animals was supplemented with Gibberelin or Gibberellic acid. The optimal concentrations here were found to be within the range of 0,0004 and 4 mg/kglw/day. The effect of improving the recovery speed of wounded animals was particularly pronounced in the range of 0,004 and 0,4 mg/kglw/day. Optimal results were achieved between 0,01 and 0,1 mg/kglw/day, such as 0,04 mg/kglw/day.

As a consequence, the invention is therefore also directed towards the use of any of the substances described above, for the preparation of a medicament for the treatment of wounded animals or the acceleration of wound healing

### References

1. Souba WW, Wilmore D. Diet and nutrition in the care of the patient with surgery, trauma, and sepsis. In Shils ME, Olson JA, Shike M, et al. Modern Nutrition in Health and Disease, 9th ed. Baltimore: Williams & Wilkins, 1999, 1589-618.
2. Breslow RA, Hallfrisch J, Guy DG, et al. The importance of dietary protein in healing pressure ulcers. J Am Geriatr Soc 1993;41 (4):357-62.
3. Tassman G, Zafran J, Zayon G. A double-blind crossover study of a plant proteolytic enzyme in oral surgery. J Dent Med 1965;20:51-4.
4. Blonstein J. Control of swelling in boxing injuries. Practitioner 1960;203:206.
5. Alvarez OM, Gilbreath RL. Effect of dietary thiamine on intermolecular collagen cross-linking during wound repair: a mechanical and biochemical assessment. J Trauma 1982; 22:20-4.
6. Aprahamian M, Dentinger A, Stock-Damge C, et al. Effects of supplemental pantothenic acid on wound healing: experimental study in rabbit. Am J Clin Nutr 1985;41:578-89.
7. Bosse MD, Axelrod AE. Wound healing in rats with biotin, pyridoxin, or riboflavin deficiencies. Proc Soc Exp Biol Med 1948;67:418-21.
8. Levine M. New concepts in the biology and biochemistry of ascorbic acid. N Engl J Med 1986;314:892-902 [review].
9. Mazzotta MY. Nutrition and wound healing. J Am Podiatr Med Assoc 1994;84:456-62 [review].
10. Mazzotta MY. Nutrition and wound healing. J Am Podiatr Med Assoc 1994;84:456-62 [review].
11. Ringsdorf WM Jr, Cheraskin E. Vitamin C and human wound healing. Oral Surg Oral Med Oral Pathol 1982;53:231-6 [review].
12. Vaxman F, Olender S, Lambert A, et al. Can the wound healing process be improved by vitamin supplementation? Experimental study on humans. Eur Surg Res 1996;28:306-14.
13. Vaxman F, Olender S, Lambert A, et al. Effect of pantothenic acid and ascorbic acid supplementation on human skin wound healing process. A double-blind, prospective and randomized trial. Eur Surg Res 1995;27:158-66.
14. Sandstead HH. Understanding zinc: Recent observations and interpretations. J Lab Clin Med 1994;124:322-7.
15. Liszewski RF. The effect of zinc on wound healing: a collective review. J Am Osteopath Assoc 1981;81:104-6 [review].
16. Pories WJ, Henzel JH, Rob CG, Strain WH. Acceleration of healing with zinc sulfate. Ann Surg 1967; 165:432-6.
17. Lansdown ABG. Zinc in the healing wound. Lancet 1996;347:706-7 [editorial].
18. Agren MS. Studies on zinc in wound healing. Acta Derm Venereol Suppl 1990;154:1-36 [review].
19. Agren MS. Zinc in wound repair. Arch Dermatol 1999:135:1273-4 [letter].
20. Cynober L. Amino acid metabolism in thermal burns. JPEN 1989;13:196.
21. De Bandt JP, Coudray-Lucas C, Lioret N, et al. A randomized controlled trial of the influence of the mode of enteral ornithine alpha-ketoglutarate administration in burn patients. J Nutr 1998; 128:563-9.
22. Cynober L. Ornithine alpha-ketoglutarate in nutritional support. Nutrition 1991;7:313-22 [review].
23. Romito RA. Early administration of enteral nutrients in critically ill patients. AACN Clin Issues 1995;6:242-56.
24. Hunt TK. Vitamin A and wound healing. J Am Acad Dermatol 1986:15:817-21 [review].
25. Hunt TK. Vitamin A and wound healing. J Am Acad Dermatol 1986; 15:817-21 [review].
26. Hunt TK, Ehrlich HP, Garcia JA, et al. Effect of vitamin A on reversing the inhibitory effect of cortisone on healing of open wounds in animals and man. Ann Surg 1969; 170:633-41.
27. Bartolomucci E. Action of vitamin E on healing of experimental wounds on parenchymatous organs. JAMA 1939; 113:1079 [abstract].
28. Ehrlich HP, Tarver H, Hunt TK. Inhibitory effects of vitamin E on collagen synthesis and wound repair. Ann Surg 1972;175:235-40.
29. Jenkins M, Alexander JW, MacMillan BG, et al. Failure of topical steroids and vitamin E to reduce postoperative scar formation following reconstructive surgery. J Burn Care Rehabil 1986; 7:309-12.
30. Bates B. Vitamin E gets an 'F' for wound healing, scarring. Family Practice News 1996;Sept 1:22.
31. Palmieri B, Gozzi G, Palmieri G. Vitamin E added silicone gel sheets for treatment of hypertrophic scars and keloids. Int J Dermatol 1995;34:506-9.
32. Rucker RB, Kosonen T, Clegg MS, et al. Copper lysyl oxidase, and extracellular matrix protein cross-linking. Am J Clin Nutr 1998;67(5 suppl):996s-1002s.
33. Tenaud I, Sainte-Marie I, Jumbou O, et al. In vitro modulation of keratinocyte wound healing integrins by zinc, copper and manganese. Br J Dermatol 1999; 140:26-34.
34. Pereira CE, Felcman J. Correlation between five minerals and the healing effect of Brazilian medicinal plants. Biol Trace Elem Res 1998;65:251-9.
35. Carlisle EM. Silicon as an essential trace element in animal nutrition. Ciba Found Symp 1986; 121:123-39.
36. Leach RM. Role of manganese in mucopolysaccharide metabolism. Fed Proc 1971;30:991.
37. Morrison LM, Murata K. Absorption, distribution, metabolism and excretion of acid mucopolysaccharides administered to animals and patients. In: Morrison LM, Schjeide OA, Meyer K. Coronary heart disease and the mucopolysaccharides (glycosaminoglycans). Springfield: Charles C. Thomas, 1974, 109-27.
38. Denuziere A, Ferrier D, Damour O, et al. Chitosan-chondroitin sulfate and chitosanhyaluronate polyelectrolyte complexes: biological properties. Biomaterials 1998;19:1275-85.
39. McCarty MF. Glucosamine for wound healing. Med Hypotheses 1996;47:273-5 [review].
40. Glade MJ. Polysulfated glycosaminoglycan accelerates net synthesis of collagen and glycosaminoglycans by arthritic equine cartilage tissues and chondrocytes. Am J Vet Res 1990;51:779-85.
41. Prudden JF, Wolarsky ER, Balassa L. The acceleration of healing. Surg Gynecol Obstet 1969;128:1321-6 [review].
42. Suyama T, Iga Y, Shirakawa H. The acceleration of wound healing with chondroitin sulfate A and its acidic hydrolysates. Jpn J Exp Med 1966;36:449-52.
43. Prudden JF, Allen J. The clinical acceleration of healing with a cartilage preparation; a controlled study. JAMA 1965;192:352-6.
44. Barbul A, Rettura G, Levenson SM, et al. Wound healing and thymotropic effects of arginine: a pituitary mechanism of action. Am J Clin Nutr 1983;37:786-94.
45. Kirk SJ, Hurson M, Regan MC, et al. Arginine stimulates wound healing and immune function in elderly human beings. Surgery 1993; 114:155-60.
46. Barbul A, Lazarou SA, Efron DT, et al. Arginine enhances wound healing and lymphocyte immune responses in humans. Surgery 1990;108:331-7.
47. Roberts PR, Black KW, Santamauro JT, Zaloga GP. Dietary peptides improve wound healing following surgery. Nutrition 1998;14;266-9.
48. Davis RH, Stewart GH, Bregman PJ. Aloe vera and the inflamed synovial pouch model. J Am Podiatr Med Assoc 1992;82(3):140-8.
49. Davis RH, Leitner MG, Russo JM, Byrne ME. Wound healing. Oral and topical activity of Aloe vera. J Am Podiatr Med Assoc 1989:79:559-62.
50. Shelton RW. Aloe vera, its chemical and therapeutic properties. Int J Dermatol 1991;30:679-83.
51. Schmidt JM, Greenspoon JS. Aloe vera dermal wound gel is associated with a delay in wound healing. Obstet Gynecol 1991;78:115-7.
52. Morisset R, Cote NG, Panisset JC, et al. Evaluation of the healing activity of hydrocotyle tincture in the treatment of wounds. Phytother Res 1987;1:117-21.
53. Kartnig T. Clinical applications of Centella asiatica (L) Urb. In Herbs, Spices, and Medicinal Plants: Recent Advances in Botany, Horticulture, and Pharmacology, vol. 3.Craker LE, Simon JE (eds). Phoenix, AZ: Oryx Press, 1986, 145-73.
54. Bossé JP, Papillon J, Frenette G, et al. Clinical study of a new antikeloid drug. Ann Plastic Surg 1979;3:13-21.
55. Shukla A, Rasik AM, Dhawan BN. Asiaticoside-induced elevation of antioxidant levels during acute wound healing. Phytotherapy Res 1999; 13:50-4.
56. Shukla A, Rasik AM, Jain GK, et al. In vitro and in vivo wound healing activity of asiaticoside isolated from Centella asiatica. J Ethnopharmacol 1999; 65: 1 -11.
57. Guillaume M, Padioleau F. Veinotonic effect, vascular protection, anti-inflammatory and free radical scavenging properties of horse chestnut extract. Arzneimittelforschung 1994;44:25-35.
58. Nasemann T. Kamillosan® therapy in dermatology. Z Allg Med 1975;25:1105-6.
59. Glowania HJ, RAulin C, Swoboda M. Effect of chamomile on wound healing-a clinical double-blind study. Z Hautkr 1987;62:162-71 [in German].
60. Glowania HJ, Raulin C, Swoboda M. The effect of chamomile on wound healing - a controlled, clinical, experimental double-blind trial. Z Hautkr 1987;62:1262-71.
61. Forest RD. Development of wound therapy from Dark Ages to the present. J Roy Soc Med 1982;75:268-73.
62. Cooper RA, Molan PC, Harding KG. Antibacterial activity of honey against strains of Staphylococcus aureas from infected wounds. J R Soc Med 1999;92:283-5.
63. Khristov G, Mladenov S. Honey in surgical practice: the antibacterial properties of honey. Khirurgiya 1961;14:937-45.
64. Obasieki-Ebor EE, Afonya TC, Onyekweli AO. Preliminary report on the antimicrobial activity of honey distillate. J Pharm Pharmacol 1983;35:748-9.
65. Vardi A, Barzilay Z, Linder N, et al. Local application of honey for treatment of neonatal postoperative wound infection. Acta Paediatr 1998;87:429-32.
66. Weiss R. Herbal Medicine. Gothenburg, Sweden: Ab Arcanum and Beaconsfield, UK: Beaconsfield Publishers Ltd, 1988, 342.
67. Blumenthal M, Busse WR, Goldberg A, et al (eds). The Complete German Commission E Monographs: Therapeutic Guide to Herbal Medicines. Austin: American Botanical Council and Boston: Integrative Medicine Communications, 1998, 83.
68. Leung A, Foster S. Encyclopedia of Common Natural Ingredients Used in Food, Drugs and Cosmetics, 2d ed. New York: John Wiley & Sons, 1996, 113-4.
69. Blumenthal M, Busse WR, Goldberg A, et al (eds). The Complete German Commission E Monographs: Therapeutic Guide to Herbal Medicines. Boston: Integrative Medicine Communications, 1998, 100.
70. Hobbs C. Echinacea: A literature review. HerbalGram 1994;30:33-48 [review].
71. Blumenthal M, Busse WR, Goldberg A, et al (eds). The Complete German Commission E Monographs: Therapeutic Guide to Herbal Medicines. Boston: Integrative Medicine Communications, 1998, 122-3.
72. Blumenthal M, Busse WR, Goldberg A, et al. The Complete German Commission E Monographs. Therapeutic Guide to Herbal Medicines. Austin, Texas: American Botanical Council, 1998, 115-6.
73. Weiss R. Herbal Medicine. Gothenburg, Sweden: Ab Arcanum and Beaconsfield, UK: Beaconsfield Publishers Ltd, 1988, 342.
74. Blumenthal M, Busse WR, Goldberg A, et al. The Complete German Commission E Monographs. Therapeutic Guide to Herbal Medicines. Austin, Texas: American Botanical Council, 1998, 231.
75. Duke JA. CRC Handbook of Medicinal Herbs. Boca Raton, FL: CRC Press, 1985, 221.
76. Blumenthal M, Busse WR, Goldberg A, et al. The Complete German Commission E Monographs. Therapeutic Guide to Herbal Medicines. Austin, Texas: American Botanical Council, 1998, 150-1.

### EXAMPLES

### Example 1 Microbiological production of a preparation containing free IAA

Azospirillum brasilence Sp7 (ATCC) was obtained as an agar culture in a culture tube. LB medium was used to grow the strain overnight at 28 °C at 175 rpm. Glycerol was added to the culture up to 10 %, mixed and divided over Nalgene creovials and frozen at - 80 °C. Stocks were stored at - 80 °C in creovials.

To prepare a seed culture of A. brasilence, one stock (1.2 to 1.8 ml) was thawed and added to 1 liter of LB medium and grown for about 20 h at 28 °C and 175 rpm to an Optical Density (OD620 nm) of about 2.5.

A 10 litre fermentor was rinsed with water and the pH electrode was calibrated. Nine litre of LB medium was prepared and 1 g/l L-Tryptophan and 0.1 g/l free IAA was added. The medium was entered into the fermentor together with 2 ml of anti foam. The fermentor was sterilised for 30 min at 121°C. After cooling down to 28 °C, the 02 probe is calibrated with N2 and 02, 0 and 100 % air saturation respectively.

The seed culture is transferred to the fermentor via a flask and tubing which are separately sterilised in an autoclave. When the addition is completed the tubing and flask are removed and the fermentation is started with the following parameters:

| | |
|---|---|
| Stirrer speed | 400 rpm |
| Temperature | 28 °C |
| Aeration PH 7 | 0.75 Nl/min |

After 15 min a sample is taken to measure the OD620 nm and check the pH. Samples are taken at certain intervals to quantify the growth of A. brasilence. When the growth rate declined extra medium was added to ensure that enough biomass was formed for the production of free IAA. It was found that the production of free IAA started when the active growth phase ended and continued for a prolonged period. The course of the free IAA concentration was followed by LC-MS. When the concentration of free IAA was at a level of about 1 g/l, the fermentation was terminated and the cells were harvested and lysed by means of a nonojet homogeniser at about 1400 bar. The remaining supernatant and the lysed cells were sterilised and spray dried to yield the desired product formulation.

### Example 2 Preparation of dog feed containing beta glucans

An amount of 5,0 gram of dried Agaricus Blazei Murill (Agaricus Farm), a natural source of beta-glucans was suspended in 100 ml of olive oil. A dog feed according to the invention was prepared by vacuum impregnating one kilogram of commercially available Royal Canin Mini Adult feed with 100 ml of the oil suspension. Control feed was prepared by vacuum impregnating the same amount of feed with only olive oil.

### Example 3 Preparation of dog feed containing plant growth hormones

An amount of the spray dried formulation as described in example 1 corresponding to 40 milligram of free IAA was suspended in 100 ml of olive oil. A dog feed according to the invention was prepared by vacuum impregnating one kilogram of commercially available Royal Canin Mini Adult feed with 100 ml of the oil suspension. Control feed was prepared by vacuum impregnating the same amount of feed with only olive oil.

### Example 4 Preparation of dog feed containing both beta glucans and plant growth hormones

An amount of 5,0 gram of dried Agaricus Blazei Murill (Agaricus Farm), a natural source of beta-glucans and an amount of the spray dried formulation as described in example 1 corresponding to 40 milligram of free IAA were suspended in 100 ml of olive oil. A dog feed according to the invention was prepared by vacuum impregnating one kilogram of commercially available Royal Canin Mini Adult feed with 100 ml of the oil suspension. Control feed was prepared by vacuum impregnating the same amount of feed with only olive oil.

### Example 5 Improving the recovery speed of dogs.

An experiment was set up to test the efficacy of the food according to the invention. In a large animal clinic, all owners of dogs that came in for a pyometra operation were asked to participate in a double-blind study. The word "pyometra" is derived from latin "pyo" meaning pus and "metra" meaning uterus. The pyometra is an abscessed, pus-filled infected uterus. Toxins and bacteria leak across the uterine walls and into the bloodstream causing life-threatening toxic effects, Without treatment death is inevitable.

Classically, the patient was an older female dog. Usually, she had finished a heat cycle in the previous 1-2 months. She had a poor appetite and may be vomiting or drinking an excessive amount of water. In the more usual "open pyometra" the cervix is open and the purulent uterine contents is able to drip out thus a smelly vaginal discharge is usually apparent.

Participants to the study were given a recovery food according to either example 2, 3 or 4. A control group received the control feed. After one year, 124 dogs had participated in the study. The veterinarians filled in a questionnaire on the recovery process of the dogs. Criteria were the speed of disappearance of fever, reoccurrence of appetite, mobility and activity of the animal and actual healing of the surgical incision. These criteria were scored on a scale ranging from 1 to 5 wherein 1 was very slow and 5 very fast. These criteria were scored 1, 2, 4 and 7 days post-surgery and 3 weeks post surgery

After one year of study, the code was broken and the results were analysed. It appeared that animals that had received feed according to examples 2 and 3 both performed significantly better than the animals that had received the control feed. After 3 weeks, no differences were found anymore between the groups. The group that received feed according to example 4 outperformed even the results of all the other groups in that their recovery was even faster. Again, after 3 weeks no significant difference was found anymore.

**Table 1**

| | Post surgery feed additives for dog food |
|---|---|
| Group 1 | 0,4 mg/kglw/day free IAA |
| Group 2 | 50 mg/kglw/day of dried ABM corresponding to 5 mg/kglw/day of 1,3-1,6 beta glucan |
| Group 3 | 0,4 mg/kglw/day free IAA plus 50 mg/kglw/day of dried ABM corresponding to 5 mg/kglw/day of 1,3 - 1,6 beta glucan |
| Group 4 (control) | none |

## Claims

1. Animal feed comprising between 0,05 and 500 milligram of beta-glucans per kilogram of feed.

2. Animal feed according to claim 1 comprising between 0,5 and 50 milligrams, preferably between 1 and 10 milligrams of beta-glucans per kilogram of feed.

3. Animal feed according to claims 1 or 2 wherein the beta glucans mainly consist of 1,3 and/or 1,6 glucans.

4. Animal feed comprising between 0,1 and 1000 milligram of phytohormones per kilogram of feed.

5. Animal feed according to claim 4 comprising between 1 and 100 milligrams, preferably between 10 and 100 milligrams of phytohormones per kilogram of feed.

6. Animal feed according to claims 4 or 5 wherein the phytohormone is free IAA.

7. Animal feed according to claims 4 or 5 wherein the phytohormone is gibberellic acid.

8. Animal feed according to claims 1 and 4.

9. Use of an animal feed according to claims 1 to 8 for improving the speed of recovery of wounded animals.

10. Use of an animal feed according to claims 1 to 8 for the preparation of a medicament for the treatment of wounded animals or the acceleration of wound healing.
